# EUROPEAN PATENT APPLICATION

(11) **EP 1 488 812 A1**
(43) Date of publication of application: **22.12.2004**
(21) Application number: 03743551.8
(22) Date of filing: 03.03.2003
(51) Int. Cl.: A61K 47/36, A61K 9/08, A61K 47/04, A61K 47/02, A61K 47/32, A61K 47/34, A61K 31/43, A61K 31/7048, A61K 31/65, A61K 31/545, A61K 31/496

(54) **LIQUID MATRIX UNDERGOING PHASE TRANSFER IN VIVO AND LIQUID ORAL PREPARATIONS**

(30) Priority: 04.03.2002 JP 2002057943
(71) Applicant: Medrx Co. Ltd., Kagawa 769-2702 (JP)
(72) Inventor: YOKOYAMA, Hideakira, Tokushima-shi, Tokushima 770-0866 (JP); HIRATA, Akihiko, Naruto-shi, Tokushima 772-0017 (JP); HAMAMOTO, Hidetoshi, Itano-gun, Tokushima 771-0207 (JP); YAMASAKI, Keiko, Higashikagawa-shi Kagawa (JP); FUJII, Takeru, Naruto-shi, Tokushima 772-0051 (JP)
(74) Representative: Müller-Boré & Partner Patentanwälte
(86) International application number: PCT/JP2003/002410
(87) International publication number: WO 2003/074086

(57) **Abstract**

It is intended to provide a liquid matrix for medicinal use in which medicine can be easily solubilized, dispersed or suspended and which can be easily swallowed because of being liquid, has favorable working properties in sterilization and so on and a high stability, also exhibits an effect of masking bitterness, and gels in vivo so as to control the release speed of the medicine, and liquid oral preparations using the same. Namely, a liquid matrix which is a liquid assistant for facilitating swallowing medicine characterized in comprising a water-soluble polymer gelling under acidic conditions, and the breaking stress of the gel is about 3.00 ×10³ N/m² or more. Liquid oral preparations have favorable slow release properties even though being a liquid.

## Description

### TECHNICAL FIELD

The present invention relates to a liquid matrix capable of regulating the release of medicine by phase transition from liquid to gel in living body, and an oral liquid preparation comprising the liquid matrix and medicine. That is, the present invention relates to a liquid matrix capable of oral administration, which can gel in stomach to exhibit a regulatory action on the rate of release of medicine.

Further, the present invention relates to a method of using an aqueous solution of a water-soluble polymer gelling under acidic conditions, as a component in a sustained-release oral liquid preparation.

### BACKGROUND ART

Generally, medicines are administered mainly via oral, and the form of the preparations including such medicines is mainly solid preparation such as powder, granule, pill, tablet and capsule.

However, easily administrable liquid preparations are preferable for infants and the elderly originally having difficulty in administration of pharmaceuticals via oral, or for patients with difficulty in swallowing of pharmaceuticals because of aging and diseases such as an aftereffect of cerebral apoplexy, cerebral contusion attributable to external injuries, cerebral palsy and Parkinson's disease.

In consideration of side effects of medicines or the like, a drug delivery system has been extensively developed in recent years as a technique of releasing medicines at desired site (small intestine, affected areas and the like). That is, the composition of a preparation is devised so as to permit the preparation to be digested in digestive tracts thereby regulating the rate of disintegration of the preparation to regulate the rate of release of the medicine. For example, Japanese Unexamined Patent Publication No. Hei 8-231435 discloses biodegradable polymeric hydrogel which releases medicines depending on biodegradation.

However, there is none of technology on liquid preparations which can regulate the rate of release of medicines. That is, solid preparations are digested generally in digestive tracts, whereby the rate of disintegration of the preparations can be utilized to regulate the rate of release of medicines. On the other hand, liquid preparations could not regulate the rate of release of medicines because the medicines are previously subjected to solubilization or the like.

For example, a pharmaceutical preparation described in Japanese Unexamined Patent Publication No. Hei 8-231435 comprises a water-soluble polymer as an essential component for regulating the release of medicine in the living body, but the preparation is in gel form so that it is not easily ingested by patients having difficulty in swallowing.

On the other hand, the oral administration of liquid preparations is the most effective administration method for infants, the elderly or patients with difficulty in swallowing, or for patients who should take a large dose of medicines. However, liquid preparations do not exhibit sustained release of medicines. Therefore, their efficacy cannot be sustained, and the frequency of administration cannot be reduced. Further, the liquid preparations often cause side effects because of good absorption of medicines and rapid increase in the concentration of medicines in blood. Accordingly, if these problems inherent in the liquid preparations can be solved, there can be brought about improvements in therapeutic effects such as reduction in the frequency of administration and reduction in side effects in addition to their inherently easy administration, thus improving the compliance of patients to achieve many medical advantages.

A large number of medicines are sparingly soluble or poor in stability upon solubilization, and have an essential problem in adding into liquid preparations. If bitter tastes of medicines and the like can be masked, resistance to administration of medicines into infants would be reduced.

Japanese Unexamined Patent Publication No. Hei 8-99885 discloses an aqueous acid-regulating composition having stable viscosity, which comprises acid-regulating agent, alginate and optionally magnesium carbonate. This composition is a gel (magnesium alginate and the like) originally having a predetermined viscosity so that swallowing thereof is not necessarily easy. Further, the gel material is separated into gel and water at high pressure or high temperatures thus making sterilization difficult, which results in a serious disadvantage in production of preparations.

A pharmaceutical preparation containing an acid-neutralizing agent (acid-regulating substance) such as an alkaline earth metal is also disclosed in Japanese Patent No. 2710375. This pharmaceutical preparation comprises pectin capable of forming coagulated gel under acidic pH, buffer and acid-neutralizing agent to form a floating raft in the stomach. By forming the raft under the acidic environment in the stomach, it is attempted to sustain efficacy and prevent the contents in the stomach from regurgitating into the esophagus. Japanese Examined Patent Publication No. SHO 46-21672 also discloses a composition comprising an acid-regulating agent and a gelling agent, and it is also attempted to sustain efficacy.

However, these prior art preparations contain a large amount of acid regulating substance, and thus the inside of the stomach is neutralized to make formation of a gel of sufficient strength impossible, thus making duration of efficacy insufficient. In addition, the preparation should be taken in a large amount, and thus it is difficult to obtain the compliance of patients in administration. When diseases such as damaged mucous membrane in the stomach advance to stomach ulcer and the like, administration of only the acid-regulating agent does not constitute fundamental treatment, and thus there is a problem that the preparation fails to serve as a therapeutic agent for stomach ulcer.

In recent years, a microorganism designated as Helicobacter pylori was isolated from mucous membrane in human stomach. It was revealed that this microorganism is a factor causing at least 80% gastritis, and is a major cause for reoccurrence of digestive ulcers, particularly duodenal ulcer. Further, it is being revealed that when infection with Helicobacter pylori is continued, contraction of stomach mucous membrane proceeds while epithelial metaplasia occurs, which leads to stomach cancer.

Accordingly, eradication of Helicobacter pylori (referred to hereinafter as "H. pylori") constitutes a fundamental treatment of gastritis, stomach ulcer, or the like. Therefore FDA in the US in 1995 recommended eradication therapy of H. pylori by using a macrolide antibiotic i.e. clarithromycin in combination with a stomach acid secretion inhibitor i.e. omeprazole or ranitidine bismuth. Five years later, that is, in September 2000, administration of three medicines i.e. a proton pump inhibitor lansoprazole combined with two antibiotics i.e. lactam-based amoxicillin and macrolide-based clarithromycin was approved in Japan as eradication therapy of H. pylori infection in stomach/duodenal ulcer. Thereafter, therapy with three medicines using the above-mentioned omeprazole was also approved in February 2002, and the eradication therapy of H. pylori is spreading as fundamental therapy for a large number of lesions such as duodenitis, erosion, erosive duodenitis and digestive ulcer.

However, antibiotics used against H. pylori are unstable to strongly acidic conditions, thus making administration in a larger amount inevitable to compensate for their antibacterial activity. On the other hand, administration of antibiotics in a larger amount easily causes side effects. Accordingly, it is important for eradication therapy of H. pylori that a proton pump inhibitor be used in combination with antibiotics thereby decreasing secretion of stomach acid to improve the degree of utilization of the antibiotics.

However, this proton pump inhibitor causes side effects such as disturbance attributable to reduction in the ability to secrete stomach acid, propagation of bacteria in the stomach, expansion of the stomach after administration, and generation of reflux esophagitis attributable to rapid secretion of stomach acid due to rebounding. Nevertheless, the amount of proton pump inhibitor used in eradication therapy of H. pylori is as twice as that usually used. Further, the presence of several % of patients for whom the proton pump inhibitor is not effective is an obstacle to use of the eradication therapy.

For stomach/duodenal ulcer, not only eradiation therapy of H. pylori but also therapy of the ulcer itself is carried out. However, conventional therapeutic agents for ulcer are those acting on ulcerous epithelial cells in mucous membrane in the stomach after they are orally ingested, absorbed from digestive tracts, carried via a portal vein into the liver where they are metabolized, and delivered with blood to the affected area. Therefore, they do not exert their therapeutic effect directly on the affected area. Accordingly, the effectiveness of the medicines is lowered, and the problem of side effects cannot be solved.

As described above, a gel preparation in consideration of medicine-releasability and a pharmaceutical preparation gelling in the stomach are known. However, there is no pharmaceutical preparation in liquid form exhibiting sustained release of medicines.

As a therapeutic method for infection with H. pylori regarded as causing gastritis or the like, a method that does not involves administering a proton pump inhibitor capable of causing severe side effects has been desired. Further, a method of treating stomach ulcer and duodenal ulcer at high level with fewer side effects has also been desired.

In view of the above, an object of the present invention is to provide a liquid matrix as a liquid pharmaceutical assistant for swallowing, which can easily solubilize, disperse or suspend medicine, is liquid to permit easy swallowing, is easily and highly operative in sterilization and the like, has an effect of masking bitter tastes of medicines and the like, and can gel in the living body to regulate the rate of release of medicines.

Another object of the present invention is to provide a liquid preparation utilizing the liquid matrix, particularly a liquid preparation effective against H. pylori for which a more excellent eradication method has been desired, as well as a liquid preparation having a therapeutic effect on stomach ulcer and duodenal ulcer.

### DISCLOSURE OF THE INVENTION

To solve the problem, the present inventors made extensive study on constituent components of pharmaceutical preparations, and found that the problem can be solved by utilizing a water-soluble polymer gelling under acidic conditions and simultaneously prescribing the breaking stress suitably after gelling, and the present invention was thereby completed.

The liquid matrix of the present invention is a liquid assistant for facilitating swallowing medicine, is characterized in comprising a water-soluble polymer gelling under acidic conditions, and the breaking stress of the gel is about 3.00×10² N/m² or more (preferably 2.00×10³ N/m² or more). Though the pharmaceutical preparation thus constituted is in liquid form, the preparation can exhibit sustained release of medicine by phase transition in the stomach after administration.

The viscosity of the liquid matrix before gelling is preferably 3.0×10⁻¹ Pa·s or less (more preferably about 1.0×10⁻¹ Pa·s or less). Because the viscosity of the preparation is low, it can be administered to patients without difficulty in swallowing, to reduce their sufferings, and thus the compliance of the patients can be easily obtained.

Preferably, the liquid matrix of the present invention comprises an insoluble salt releasing polyvalent metallic cation under acidic conditions, and the insoluble salt is preferably alkaline earth metal salt of inorganic acid. Preferably, the water-soluble polymer contained in the liquid matrix has carboxyl group and/or sulfonic acid group (which may include both carboxylic acid and sulfonic acid group) in the chemical structure thereof, and specifically alginate, pectin, the combination of alginic acid or alginate and pectin, gellan gum, or the combination of gellan gum and pectin is preferable. By using these water-soluble polymers, a gel having breaking stress higher than a predetermined value can thereby be reliably formed in the stomach to achieve excellent sustained release of medicine.

The oral liquid preparation according to the present invention is characterized in comprising the liquid matrix and medicine, and has the characteristics of the liquid matrix. That is, the preparation is characterized in that it can be swallowed easily due to liquid and by masking of the bitter taste of the medicine, and gels in the stomach to exhibit sustained release of the medicine.

The medicine is preferably the one showing an anti-H. pylori activity. Unlike the conventional therapeutic method of eradicating H. pylori, the oral liquid preparation of the invention does not necessitate proton pump inhibitor, and can reduce the amount of antibiotics which should be administered in the prior art in a larger amount and easily leads side effects, while this oral liquid preparation can sustain the pharmacological effect of the medicine.

Such medicine showing anti-H.pylori activity includes at least one member selected from the group consisting of penicillin antibiotics, macrolide antibiotics, tetracycline antibiotics, cepham antibiotics, and pyridonecarboxylic acid synthetic antibacterial agents. Further, at least one member selected from the group consisting of amoxicillin, clarithromycin, roxithromycin, minocycline hydrochloride, cephaclor, cephalexin, ofloxacin, tosufloxacin tosylate, and levofloxacin can be exemplified. Though these medicines exhibit high anti-H.pylori activity, they are unstable to strongly acidic conditions, and thus fail to exhibit their antibacterial activity efficiently by the conventional administration method. Given an oral liquid preparation utilizing the liquid matrix according to the present invention, however, the amount of these medicines to administer can be reduced to such a range that side effects are not exhibited, and the antibacterial activity can be exhibited.

The oral liquid preparation has the sustained release of the medicine, that is, the most distinctive feature of the liquid matrix of the present invention, and is significantly superior in this feature to conventional liquid preparations.

The medicines are preferably those having a therapeutic effect on stomach ulcer or duodenal ulcer. According to liquid preparations having such medicine, the therapeutic effect is high and side effects can be reduced, because the medicine can be sustainedly released to act directly on the affected area in addition to an affected area can be covered and protected with the gel by transformation of the liquid matrix to gel in the stomach. In treatment of duodenal ulcer, the liquid preparation of the present invention having gelled in the pyloric vestibule of the stomach can sustainedly release the medicine to permit it to act sufficiently from the pyloric region to the duodenum. Further, an affected area of the duodenum is covered with the gel preparation after gradual transfer to the duodenum by peristalsis, and the medicine is sustainedly released to the affected area. Accordingly, the liquid preparation is very useful as a therapeutic agent for stomach ulcer and duodenal ulcer.

The medicines having a therapeutic effect on stomach ulcer or duodenal ulcer are preferably those having effect of promoting protection factor. This is because experiments described later have revealed that in the liquid preparation utilizing the liquid matrix according to the present invention, medicines of protection factor promoting type have a higher therapeutic effect.

The medicines having an effect of promoting protection factor are preferably prostaglandin or derivatives thereof. This is because prostaglandin and derivatives thereof have an effect of increasing a blood stream in mucous membrane and a promoting effect on the ability to secrete viscous fluid, and have actual results as remedies for stomach ulcer and duodenal ulcer.

The oral liquid preparation comprises the liquid matrix of the present invention as a component, and thus naturally has an ability to release medicines sustainedly, and in this respect, this preparation is significantly superior to the conventional liquid preparations.

The method according to the present invention is characterized in that an aqueous solution of a water-soluble polymer gelling under acidic conditions is used as a component in the sustained-release oral liquid preparation. The method of the invention also has the characteristics of the liquid matrix of the invention.

The most distinctive feature of the liquid matrix according to the present invention is that it is liquid in an ordinary state, and thus a relatively large amount of medicine may be added to the matrix, and the liquid matrix can be easily swallowed, while after oral administration, it can gel in the stomach to achieve sustained release of the medicine, and its efficacy can be maintained. That is, when the liquid matrix according to the present invention enters in liquid form through the esophagus into the stomach, the water-soluble polymer as one component gels by the strong acidity of stomach acid.

Unlike the conventional liquid preparation which is sent immediately to the small intestine where the medicine as an active ingredient is absorbed, upon reaching the stomach, so that the blood concentration of the medicine is rapidly increased and then rapidly decreased, the oral liquid preparation according to the present invention is sent gradually to the small intestine by peristalsis of the stomach, whereby the medicine is continuously absorbed and the efficacy is exhibited continuously.

Hereinafter, embodiments of the present invention exhibiting such characteristics, and the effect thereof, are described.

The "water-soluble polymer gelling under acidic conditions" is not particularly limited insofar as it is pharmacologically acceptable and gels with stomach acid. The polymer can be exemplified by alginic acid or a salt thereof, pectin, gellan gum or a combination thereof. A water-soluble polymer which does not gel by themselves under acidic conditions, but gels under acidic conditions when used in combination with gellan gum and the like, can also be used. Such water-soluble polymer includes arabic gum, carrageenan, tamarind seed gum, guar gum, xanthane gum, curdlan, hyaluronic acid and locust bean gum. The combination thereof is preferably a combination of gellan gum and pectin, carrageenan or locust bean gum. A combination of alginic acid or a salt thereof and locust bean gum is also preferable.

For forming a gel of high physical strength to exhibit excellent sustained release, the water-soluble polymer is preferably crosslinked with polyvalent metallic cations. The water-soluble polymer used therein is not particularly limited insofar as it is water-soluble in an ordinary state and gels upon addition of polyvalent metallic cations, and the water-soluble polymer should be pharmacologically acceptable. Such water-soluble polymer includes, for example, alginates such as sodium alginate and propylene glycol alginate; vinyl polymers such as carboxyvinyl polymer, sodium polyacrylate and polyvinyl alcohol; cellulose derivatives such as carboxymethyl cellulose gum, carboxymethyl cellulose and carboxyethyl cellulose; plant polysaccharides such as konjak mannan, pectin, carrageenan and guar gum; microbial polysaccharides such as dextran; and combinations of two or more thereof.

The mechanism by which these water-soluble polymers are gelled with polyvalent metallic cations is not completely clarified, but an egg box model is proposed wherein two carboxyl groups in the polymer are crosslinked via a coordinate bond with one divalent cation to gel the water-soluble polymer. Accordingly, when divalent cations are present in a sufficient amount relative to carboxyl groups in the polymer, a gel of high density of crosslinkage is obtained, while when the amount of divalent cations is limited at a low level, a gel of low density of crosslinkage is obtained. In view of the above, it is preferable that after the amount of carboxyl groups in the "water-soluble polymer gelling by polyvalent metallic cations" is grasped, the contents of the "insoluble salt releasing polyvalent metallic cations under acidic conditions" and the "water-soluble polymer gelling by polyvalent metallic cations" are determined, whereby the properties of gel formed in the stomach can be controlled and the sustained release of medicines can be regulated. In consideration of the egg box model, the "water-soluble polymer gelling by polyvalent metallic cations" preferably has carboxyl group or sulfonic acid group, or both of these groups in the structure thereof.

Preferable among those described above are alginic acid, alginate, pectin, a combination of alginic acid or alginate and pectin, gellan gum, a combination of pectin and gellan gum, a combination of carrageenan and locust bean gum, and a combination of gellan gum and arabic gum, most preferably sodium alginate and gellan gum. When a gel of high physical strength is required, pectin is LM pectin with a low degree of methylation, and when so high physical strength is not desired, HM pectin may also be used.

The physical strength of the gel can be regulated not only by selecting the water-soluble polymer or by adding polyvalent metallic cations but also by a combination of two or more water-soluble polymers. For example, alginic acid or alginate and pectin, gellan gum and pectin, or carrageenan and locust bean gum are combined and their compounding ratio is devised, whereby a gel of high physical strength can be formed in living body. The liquid matrix transformed into a gel of high physical strength in living body is not immediately disintegrated in the stomach, is transferred to the small intestine by peristalsis of the stomach, and then gradually disintegrated in the small intestine and thereafter to exhibit excellent sustained release of the medicine.

Further, the liquid matrix itself and the gel can be modified by adding water-soluble polymers, for example polyethylene glycol, polyethylene glycol/polypropylene glycol block copolymer and the like; animal proteins such as gelatin, casein, and collagen; or starch such as soluble starch, and methyl starch.

When the liquid matrix of the present invention is applied to an anti-H. pylori preparation, the active medicine remains for a longer time in the stomach than in merely drinking the medicine with water, and thus the original activity of the medicine can be exhibited more effectively than by conventional administration of 3 medicines. Further, when the liquid matrix of the present invention gels in the stomach, it could be crosslinked with a mucous layer of the stomach to exhibit efficacy for a long time. That is, H. pylori itself is weak to strong acidity, and is thus protected by the surface mucous membrane of the stomach, and because the mucous layer is coherent to the gel, the medicine contained in the anti-H. pylori oral liquid preparation of the invention can exhibit efficacy without undergoing the influence of stomach acid.

As described above, the water-soluble polymer are selected and combined, metallic anions are added or not added, and the amount thereof is regulated, whereby a gel having various properties by which the gel is gradually disintegrated in the stomach or is stable in the stomach and disintegrated in the small intestine can be formed in the living body to achieve sustained release for exhibiting the activity of the medicine to the maximum degree.

The physical strength of the gel formed from the liquid matrix of the present invention should have a breaking stress of about 3.00×10² N/m². This is because when the breaking stress is less than this strength, the sustained release of the medicine cannot be maintained. The "about 3.00×10² N/m² or more" refers to a numerical value which upon being rounded off, becomes at least 3.00×10² N/m², and refers specifically to a numerical value of 2.95×10² N/m² or more. The breaking stress is preferably 8.70×10² N/m² or more, most preferably 2.00×10³ N/m² or more.

The relationship of the concentration of the water-soluble polymer contained in the matrix of the present invention with the viscosity of the liquid matrix or the physical strength of the gel is significantly varied depending on the properties of the water-soluble polymer, and thus the concentration is not particularly limited. However, it is recommended that the concentration be generally 0.01 to 10% by mass.

The "insoluble salt releasing polyvalent metallic cations under acidic conditions" used in the present invention is insoluble or sparingly soluble in an aqueous neutral or weakly basic solution, but is dissolved under acidic conditions to release polyvalent metallic cations. The insoluble salt is not particularly limited insofar it is pharmacologically acceptable, but a salt containing a divalent or trivalent metallic cation is preferable. Such insoluble salt includes alkaline earth metal salts of inorganic acid such as barium carbonate, barium sulfate, strontium carbonate, calcium carbonate, calcium phosphate, magnesium dihydrogen phosphate, magnesium aluminate silicate, magnesium aluminate metasilicate, calcium hydrogen phosphate anhydride, and calcium hydrogen phosphate; light metal salts of inorganic acid such as synthetic aluminum silicate and aluminum phosphate; hydroxides such as magnesium hydroxide, magnesium alumina hydroxide, aluminum hydroxide, and dry aluminum hydroxide gel; alkali earth metal oxides such as magnesium oxide; synthetic hydrotalcite; dihydroxyaluminum aminoacetate and dihydroxyaluminum aminoacetate; and sucrose ester aluminum salts.

The term "insoluble" means that generally at least 10,000 g water is required to dissolve 1 g sample, and "sparingly soluble" means that 1,000 to 10,000 g water is required to dissolve 1 g sample. The solubility of the insoluble salts can be higher than the above range insofar as the object of the present invention can be achieved.

The content of the "insoluble salt" is preferably about 10% by mass or less, more preferably about 5% by mass or less, relative to the total weight of the liquid matrix or the preparation. A number after the term "about" refers to a number which is previously rounded off; for example, "about 10% by mass or less" specifically refers to "less than 10.5% by mass". The lower limit of the "insoluble salt" contained is determined by the amount of the "water-soluble polymer gelling by metallic cations" contained and by a desired degree of crosslinkage of gel in the stomach.

The molar ratio of the multivalent metallic cation possessed by the insoluble salt to the carboxyl group or sulfonic acid group in the structure of the water-soluble polymer is preferably 1 to 10, more preferably 3 to 5.

The liquid matrix according to the present invention is prepared by dissolving the "water-soluble polymer gelling under acidic conditions" in water.

The "water" used herein is not particularly limited insofar as it is a pharmacologically acceptable aqueous solvent, the water is preferably distilled water or physiological saline, particularly preferably distilled water.

When the "insoluble salts releasing polyvalent metallic cations under acidic conditions" is to be contained in the liquid matrix according to the present invention, it is preferable that the water-soluble polymer is first dissolved in water, and then the insoluble salt is added thereto. This is because the insoluble salt is insoluble or sparingly soluble in water. To disperse the insoluble salt uniformly in the liquid matrix, sonication or the like may be conducted. The prepared liquid matrix is preferably subjected to sterilization treatment such as high-pressure sterilization for pharmacological use.

When the insoluble salt is to be contained, the pH of the liquid matrix according to the present invention should be neutral or basic so as not to dissolve the insoluble salt, or should be neutral or weakly basic in consideration of the stability of medicine added. Further, when the medicine added contains polyvalent metallic cations or shows acidity, the pH of the liquid matrix should be regulated in advance in consideration of the properties of the medicine. The acid or base used in this regulation is not particularly limited insofar as it is pharmacologically acceptable, but a solution of hydrochloric acid or sodium hydroxide is preferably used.

The liquid matrix according to the present invention may be blended depending on the intended object with salts, surfactants, coloring matters, flavoring ingredient, acidic tasting materials, sweeteners, preservatives (parabene, sodium benzoate and the like), as long as they are pharmaceutically acceptable. The scope of the present invention is not limited thereto.

The viscosity of the prepared liquid matrix is preferably 3.0×10⁻¹ Pa·s or less, more preferably about 2.0×10⁻¹ Pa·s or less, still more preferably about 1.0×10⁻¹ Pa·s or less, further more preferably 1.0×10⁻¹ Pa·s or less. This is because when the viscosity is higher than the above range, infants or patients with difficulty in swallowing suffer from swallowing of the liquid matrix of the present invention. Accordingly, it is recommended that the viscosity of the liquid matrix be lower for patients having more difficulty in swallowing. The "about 2.0×10⁻¹ Pa·s or less" refers to a number which upon being rounded off, becomes 2.0×10⁻¹ Pa·s or less, and refers specifically to a number less than 2.5×10⁻¹ Pa·s. Similarly, "about 1.0×10⁻¹ Pa·s or less" refers to a number of less than 1.5×10⁻¹ Pa.s.

The "medicine" incorporated together with the liquid matrix into the oral liquid preparation of the present invention is not particularly limited, and not only one medicine but also two or more medicines may be incorporated. A soluble and stable medicine may be mixed as it is, and a sparingly soluble medicine may be formed into a conjugate with cyclodextrin in order to solubilize and stabilize the medicine. Alternatively, a sparingly soluble medicine may be dispersed or suspended as it is. Because the liquid matrix of the present invention has a dispersing effect on the medicine so that even if the medicine is insoluble or sparingly soluble, the medicine can be dispersed therein and prevented from being precipitated.

The "medicine" incorporated into the oral liquid preparation of the present invention includes agents for the nervous system such as hypnotic analgesics, antianxiety agents, anti-epilepsy agents, antipyretic sedative antiphlogistics, anti-Parkinson agents, agents for psychosis and neurological disorders, and agents for the cold; agents for circulation organs such as agents for arrhythm, diuretics, blood pressure depressants, vasoconstrictors, vasodilators, and agents for hyperlipemia; agents for respiratory organs such as respiratory stimulant, cough suppressant, expectorants, anti-cough expectorants, and bronchodilator; agents for digestive organs such as antidiarrhoic, antiflatulent, medicines for digestive ulcer, medicines for stomach digestion, laxative, medicines for gallbladder, and medicines for stomach ulcer and duodenal ulcer; various hormones; medicines for urinogenital organs and anus such as medicines for urinary organs, medicines for generative organs, and uterus shrinking medicines; metabolic pharmaceutical preparations such as vitamins, revitalizer, medicines for blood and body fluid, medicines for hepatic diseases, antidotes, medicines for habitual poisoning, gouty medicines, enzymes preparations, diabetic medicines, cell activators, antitumor agents, medicines for allergies, antibiotics including anti-pylori agents, medicines for chemotherapy, biological preparations, parasiticides, opium alkaloid drugs, and non-alkaloid drugs. The medicines used in the invention are not limited in the above.

When dl-methylephedrine hydrochloride, noscapine, dextromethorphan hydrobromide, dihydrocodeine phosphate or a dihydrocodeine/ephedrine blend used in therapy of infant asthma is incorporated as the medicine into the liquid matrix of the present invention, the activity of the medicine can be maintained and the frequency of administration can be reduced, and thus it is very effective for treatment of infants.

In the therapy of patients with terminal cancer, morphine preparations or codeine preparations are used singly or in combination for the purpose of relieving cancerous pain, and these are solid pharmaceutical preparations. Easily swallowed preparations are desired for patients with terminal cancer, but when these preparations are used as liquid, the preparations cannot be durable. When the liquid matrix of the present invention is used, the preparations can be administered as durable pain reducers into the patients.

Further, when the preparation of the invention is used for stomach ulcer or gastritis, anti-inflammatory ulcer agents can be added. Such agents include methyl thionine preparations, azulene preparations, herb extracts, aceglutamide, aldioxa, urogastron, ecabet sodium, cetraxate hydrochloride, pirenzepine hydrochloride, benexate hydrochloride, enprostyl, ornoprostil, gefarnate, scralfate, sulpiride, sofalcon, teprenone, troxipide, plaunotol, proglumide, polaprezinc, irsogladine maleate and misoprostol.

Regardless of the foregoing, the oral liquid preparation blended with the medicine having an anti-H. pylori activity exhibits a particularly high effect. That is, the oral liquid preparation of the present invention is "liquid", and can thus be easily administered to the elderly or persons having difficulty in swallowing. After ingestion into the stomach, it spreads to every hole and corner of stomach wall having a complicated villous structure, and enters into crypts to which a gel preparation or the like hardly enters, followed by gelling with the strong acidity of hydrochloric acid as a major component of stomach acid secreted from the fundic gland. Accordingly, the liquid preparation of the present invention can protect the stomach mucous membrane throughout the stomach and can sustainably release the efficacy component to an affected area, and is thus very effective in treatment of gastritis and stomach ulcer. Further, when the liquid matrix of the present invention gels in the stomach, it could be crosslinked with a mucous layer of the stomach to exhibit efficacy for a long time. That is, H. pylori itself is weak to strong acid and is protected by the surface mucous membrane of the stomach, and because the gel is coherent to this mucous layer, the medicine contained in the anti-H. pylori oral liquid preparation of the present invention can exhibit the efficacy without undergoing the influence of stomach acid.

The "medicine exhibiting an anti-H. pylori activity" is not particularly limited insofar as it is effective against H. pylori and pharmacologically acceptable. The examples thereof include penicillin antibiotics such as amoxicillin, macrolide antibiotics such as clarithromycin, roxithromycin, and azithromycin, tetracycline antibiotics such as minocycline hydrochloride, cepham antibiotics such as cephachlor, cephalexin, and cefdinir, and pyridonecarboxylic acid synthetic antibacterial agents such as ofloxancin, tosufloxacin tosylate, levofloxancin, norfloxacin, and gatifloxacin, and metronidazole, from which at least one member can be selected and used.

Particularly preferable among these are amoxicillin, clarithromycin, roxithromycin, minocycline hydrochloride, cephachlor, cephalexin, ofloxancin, tosufloxacin tosylate, and levofloxancin, and it is recommended that two or more thereof are simultaneously administered. The medicines are not particularly limited insofar as their antibacterial action on H. pylori is strong.

Regardless of the foregoing, the oral liquid preparation blended with the "medicine" having a therapeutic effect on stomach ulcer or duodenal ulcer exhibits a particularly high effect. This is because unlike the conventional anti-ulcer agent, the medicine can be released gradually and directly to an affected area, and thus the utilization of the medicine is high, and side effects can be reduced.

The "medicine having a therapeutic effect on stomach ulcer or duodenal ulcer" can be classified into medicines having effect of "inhibiting attack factor" mainly inhibiting secretion of stomach acid and inhibiting an attack factor causing ulcer and "promoting protection factor" mainly having a protective effect on stomach mucous membrane and an effect of promoting repair of ulcerous sites. The medicine used in the present invention is not particularly limited insofar as it is acceptable under the Pharmaceutical Affairs Law. However, acid regulators such as aluminum hydroxide and sodium hydrogen carbonate directly neutralizing stomach acid are preferably not used. This is because the liquid matrix according to the present invention may be prevented from gelling, to fail to exhibit the sustained release of the medicine.

The medicine having effect of "inhibiting attack factor" and therapeutic effect on stomach ulcer or duodenal ulcer includes, for example, proton pump inhibitors such as omeprazole, lansoprazole, and rabeprazole; H2 blockers such as cimetidine, ranitidne, and famotidine; selective muscaline receptor antagonists such as pirenzepine; and anti-gastrin agents such as proglumide, secretin, and urogastrone. The medicine having effect of "promoting protection factor" and therapeutic effect on stomach ulcer or duodenal ulcer includes, for example, ulcerous lesion protecting agents such as sucralfate, and azulene; tissue repair promoters such as aldioxa, gefarnate, ecabet sodium, and L-glutamine; mucous fluid production/secretion promoters such as teprenone, plaunotol, ornoprostil, enprostil, and rebamipide; stomach mucous membrane finite circulation improvers such as cetraxate hydrochloride, sofalcon, sulpiride, and benexate betadex hydrochloride; local anti-inflammatory agents such as azulene sulfonate sodium; and prostaglandin such as PGE1 (alprostadil alphadex) and PGE2.

Among the medicine having effect of "inhibiting attack factor" and "promoting protection factor", the medicine having effect of "promoting protection factor" is more preferable. This is because when stomach acid secretion itself is inhibited, the protecting ability of stomach acid is reduced, and thus infection of bacteria may occur.

The "medicine having herapeutic effect on stomach ulcer or duodenal ulcer" is preferably prostaglandin or a derivative thereof. This is because the prostaglandin has results as a medicine for digestive tract ulcer, and the effect of the liquid preparation of the invention containing the prostaglandin is proven by the Examples described later.

The time of blending the medicine with the liquid matrix of the present invention is not particularly limited. The medicine may be mixed just before administration. For example, medicines, of which stability is deteriorated upon dissolution in water, are mixed preferably just before administration.

Even if the medicine is incorporated into the liquid matrix of the present invention, the viscosity thereof before and after incorporation is hardly changed. The viscosity after incorporation, just as the viscosity of the liquid matrix, is preferably 3.0×10⁻¹ Pa·s or less, more preferably about 2.0×10⁻¹ Pa·s or less, still more preferably about 1.0×10⁻¹ Pa·s or less, further more preferably 1.0×10⁻¹ Pa·s or less. Sterilization treatment such as high-pressure sterilization may be conducted after the medicine was blended.

The present invention was constituted as described above, and the liquid matrix of the present invention has an effect of masking bitter tastes of medicine and the like, and is thus useful as a swallowing assistant for the medicine, and exhibits the sustained release of the medicine by gelling in the living body.

Accordingly, the liquid oral preparation having the liquid matrix of the present invention as the component has the above-described effect, is easily swallowed and exhibits sustained release of the medicine, and thus even if it contains medicine having high side effects as component, the efficacy can be sustained without rapidly increasing its blood concentration, and the frequency of administration can be reduced.

As the oral liquid preparation of the present invention, the one containing an anti-H pylori agent is highly effective. This is because in eradication therapy of H. pylori, for which no effective therapeutic method has been established, the amount of the antibiotic blended can be reduced without using a proton pump inhibitor, and the side effect can thereby be inhibited. Further, the liquid preparation containing medicine having therapeutic effect on stomach ulcer or duodenal ulcer can directly act on ulcerous legions, thus exhibiting a very high therapeutic effect thereon.

### BRIEF DESCRIPTION OF THE DRAWINGS

Fig. 1 shows the relationship between the strength (breaking stress) of gel and release of riboflavin from the gel.
Fig. 2 shows sustained release of riboflavin from the oral liquid preparation of the present invention.
Fig. 3 shows sustained release of acetaminophen from the oral liquid preparation of the present invention.
Fig. 4 shows autoclaved states of the liquid matrix of the present invention and a comparative example.
Fig. 5 shows sustained release of riboflavin from the oral liquid preparation of the present invention containing two kinds of water-soluble polymers.
Fig. 6 shows sustained release of riboflavin from the oral liquid preparation of the present invention.

### BEST MODE FOR CARRYING OUT THE INVENTION

Hereinafter, the present invention is described in more detail by reference to the Examples and Preparation Examples, but the scope of the present invention is not limited thereto.

### Example 1

Sodium alginate was added to distilled water to prepare 10 ml aqueous solutions of a water-soluble polymer at various concentrations, and various amounts of calcium carbonate or calcium phosphate were added as the insoluble salt to prepare liquid matrixes. 5 ml of in the Japanese Pharmacopoeia Disintegration Test Liquid 1 was added to each liquid matrix, and a reproduction test was conducted where the liquid matrix of the present invention was assumed to be introduced into the stomach, and the state of gelling was observed. The results are shown in Tables 1 and 2.

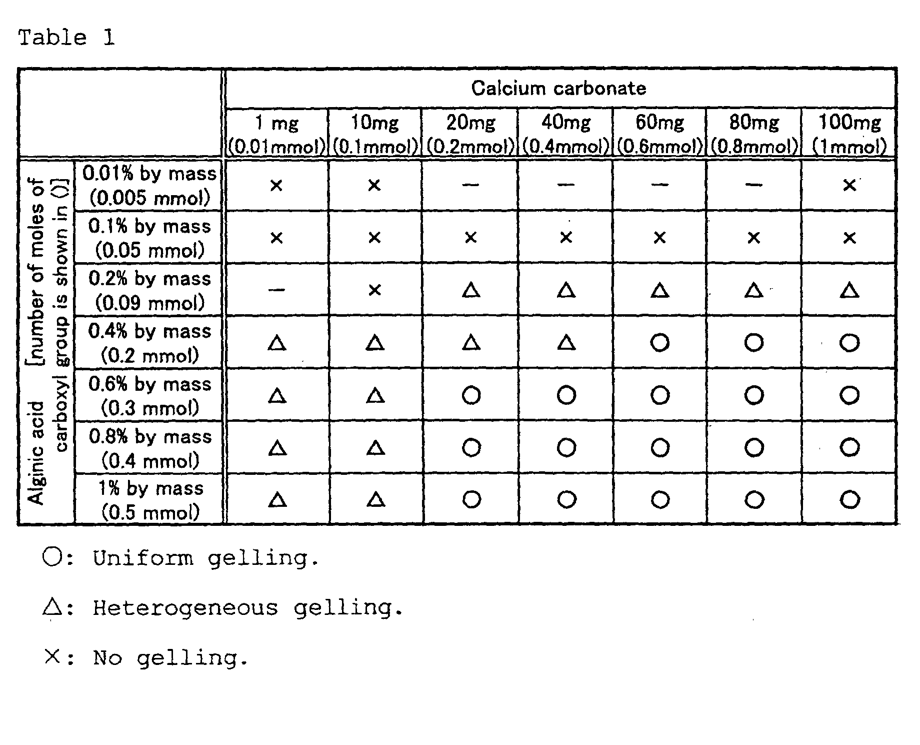

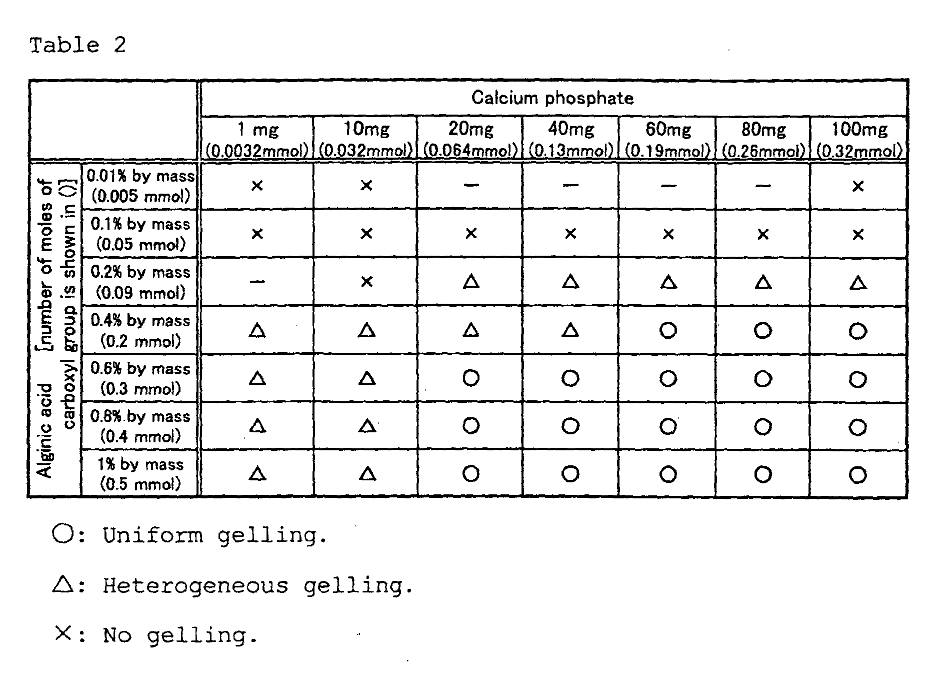

Components constituting in sodium alginate are sodium β-D-mannuronate and sodium α-L-gluronate, and both the molecular weights of the sodium salts of these such sugar acids are about 217 and one carboxyl group is possessed by one sugar. Therefore, the number of moles of carboxyl groups in sodium alginate at each concentration can be roughly calculated.

From the results in Tables 1 and 2, it was found that when the insoluble salt releasing metallic cations is contained to uniformly gel the liquid matrix of the present invention, the molar ratio of the metallic cation possessed by the insoluble salt to the carboxyl group or sulfonic acid group in the structure of water-soluble polymer is preferably 1 or more.

### Example 2

Two kinds of water-soluble polymers were mixed to prepare liquid preparations to transform them into gels having varying gel strength (gel shear stress), and a test of sustained release of medicine was conducted.

κ -Carrageenan and locust bean gum were added in a varying mixing ratio as the water-soluble polymers to 100 ml distilled water and stirred sufficiently to prepare a plurality of liquid matrixes to be transformed into gels having varying gel strength. Further, riboflavin was added at a final concentration of 0.02% and adjusted to pH 7.4 to prepare liquid preparations.

1 ml of this riboflavin-liquid matrix was dropped along a tube wall into a tube containing 30 ml Japanese Pharmacopoeia Disintegration Test Liquid 1, and a reproduction test was conducted where the riboflavin-liquid matrix was assumed to be introduced into the stomach. The riboflavin-liquid matrix gelled upon contacting with the Disintegration Test Liquid 1. The strength of each gel was determined in terms of breaking stress (N/m²) by Yamaden Creep Meter HC2-3805.

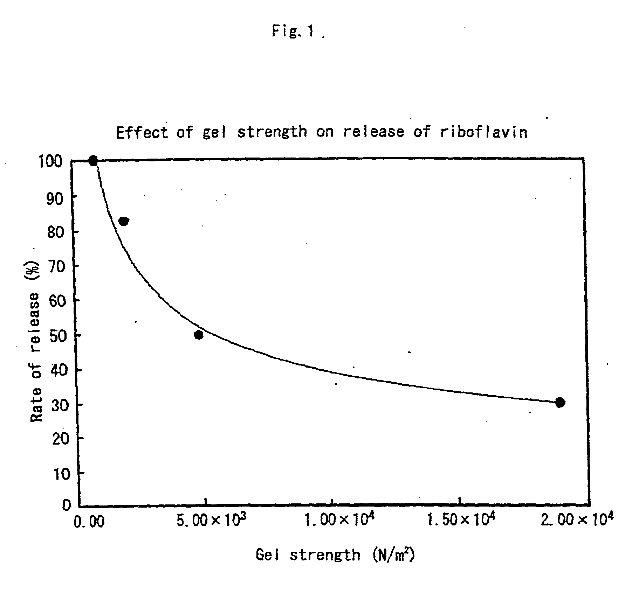

The gel was completely introduced into the Disintegration Test Liquid 1 and then stirred at 37°C for 120 minutes, and the release of riboflavin was measured. The riboflavin thus released was calorimetrically quantified by measuring absorbance at 445 nm according to C-2660 to 2667 in Manual of the Japanese Pharmacopoeia 13th ed. (1996) published by Hirokawa Shoten. Assuming that the amount of riboflavin in the original gel was 100, the amount (%) of riboflavin released to the Disintegration Test Liquid 1 was calculated. The relationship between the gel strength and the degree of release of riboflavin is shown in Fig. 1.

As shown in Fig. 1, riboflavin is strongly maintained in the gel when the strength of the gel is high, and the medical is sustainedly released. That is, the gel can be evaluated as having sustained releasability when the medicine remains in the gel even after stirring at 37°C for 120 minutes. On the other hand, when the gel strength is low, riboflavin is immediately released, and the gel cannot show sustained release. That is, usually, when gel not showing sustained release is dipped in the Disintegration Test Liquid 1 at 37°C, the gel immediately releases medicine, and the shape of the gel is lost in about 20 minutes. In this experiment, the gel was regarded as having the minimum strength for attaining sustained release when 10% or more of the medicine remained in the gel after dipping in the Disintegration Test Liquid 1 at 37°C for 120 minutes. From the result, the rupture stress was about 3. 00×10² N/m².

### Example 3

1 g of sodium alginate or 1 g of LM pectin was added to and completely dissolved in 100 ml distilled water, and then 1 g of calcium carbonate was added. The mixture was adequately stirred to make two liquid matrix. To this solution, 100 mg of riboflavin was added and dissolved to adjust the pH to 7.4.

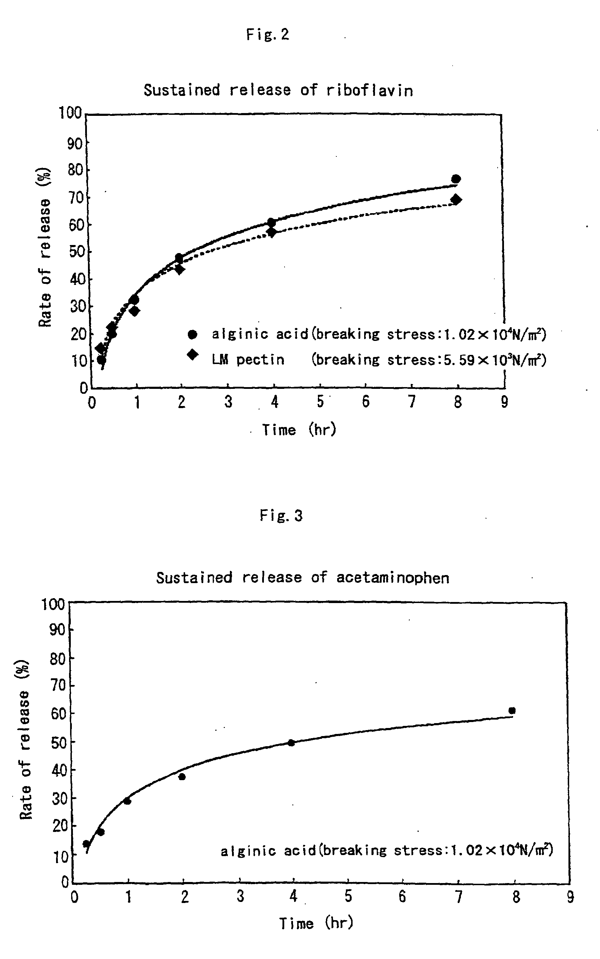

1 ml of this riboflavin-liquid matrix was gelled by treating it in the same manner as in Example 2. When the strength of each gel was measured in the same manner as in Example 2, the strengths of the gels formed from 1% sodium alginate and 1% LM pectin were 1.02×10⁴ N/m² and 5.59×10³ N/m², respectively. The amount of riboflavin released from the gel was measured. Assuming that the amount of riboflavin in the original gel was 100, the amount (%) of riboflavin released to the Disintegration Test Liquid 1 was calculated. The results are shown in Fig. 2.

As shown in Fig. 2, it was found that in both the case where alginic acid and LM-pectin was used as the water-soluble polymer, riboflavin was sustainedly released without rapidly being released to the test solution. Accordingly, it was proved that the oral liquid preparation of the present invention exhibits excellent sustained releasability.

### Example 4

1 g of sodium alginate was added to 100 ml of distilled water and dissolved completely, and 1 g calcium carbonate was added thereto. The mixture was stirred sufficiently to prepare a liquid matrix. 10 mg of acetaminophen (Yoshitomi Fine Kagaku Co., Ltd.) was added thereto to adjust pH to 7.4.

1 ml of this acetaminophen-liquid matrix was dropped into 100 ml of the Japanese Pharmacopoeia Disintegration Test Liquid 1, and a reproduction test was conducted where the acetaminophen-liquid matrix was assumed to be introduced into the stomach. The dropped acetaminophen-liquid matrix was immediately gelled. The strength of the gel was 1.02×10⁴ N/m². Release of the acetaminophen from this gel was measured. The released acetaminophen was calorimetrically quantified by measuring absorbance at 244 nm according to C-69 to C-73 in Manual of the Japanese Pharmacopoeia, 13th ed., Hirokawa Shoten (1996). Assuming that the amount of acetaminophen in the original gel was 100, the amount (%) of acetaminophen released into Disintegration Test Liquid 1 was calculated. The results are shown in Fig. 3.

As shown in Fig. 3, it was revealed that when the acetaminophen-liquid matrix was introduced into the stomach, the water-soluble polymer was gelled, and acetaminophen was sustainedly released. Accordingly, it was evidenced that the oral liquid preparation of the present invention shows excellent sustained releasability.

### Example 5

The acetaminophen-liquid matrix prepared in Example 4 was sterilized and treated at high pressure at 121°C for 30 minutes, followed by being left to reduce the pressure, whereby a test sample was obtained.

As a comparative example, an acid-regulating composition having stable viscosity was prepared by referring to a description of Japanese Unexamined Patent Publication No. Hei 8-99885. That is, 2.5 g of sodium alginate and 28 g of aluminum hydroxide/magnesium carbonate gel (56% Al₂O₃, 4% MgO) were added to about 75 ml of pure water and stirred at about 80°C for 30 minutes. This heated reaction mixture was rapidly mixed under stirring with 400 ml aqueous suspension of 35 g magnesium carbonate kept at about 25°C. 47.5 g of sodium alginate was added to this mixture, and 1 g of xanthane gum, 15 g of calcium carbonate and 10 g of potassium hydrogen carbonate were added respectively thereto, and the mixture was adjusted to 1 L with pure water. The comparative product thus produced was sterilized at high pressure in the same manner as for the acetaminophen-liquid matrix of the present invention.

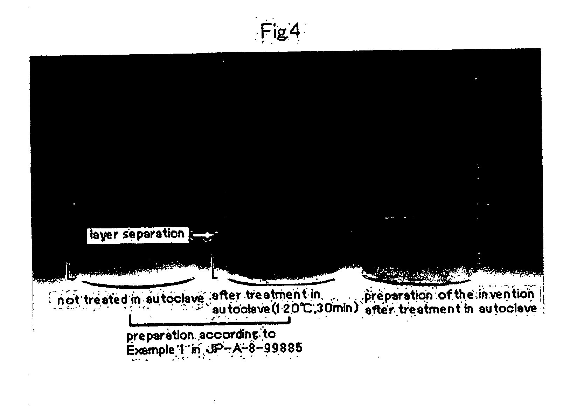

The states of both the samples after high-pressure sterilization are shown in Fig. 4.

As shown in Fig. 4, the oral liquid preparation of the present invention was maintained stably even after high-pressure sterilization, while the comparative product was separated completely into an aqueous layer and a gel layer. It was thus found that the operativeness for sterilization of the oral liquid preparation of the present invention is extremely high.

### Example 6

In the same manner as in Example 2, two kinds of water-soluble polymers were added to and completely dissolved in 100 ml distilled water to form (i) 0.5% alginic acid-0.5% pectin and (ii) 1% alginic acid-1% pectin respectively, and then 1 g of calcium phosphate was added thereto. The mixture was sufficiently stirred to prepare liquid matrixes. 100 mg of riboflavin was added to and dissolved in each solution, to adjust the pH to 7.4.

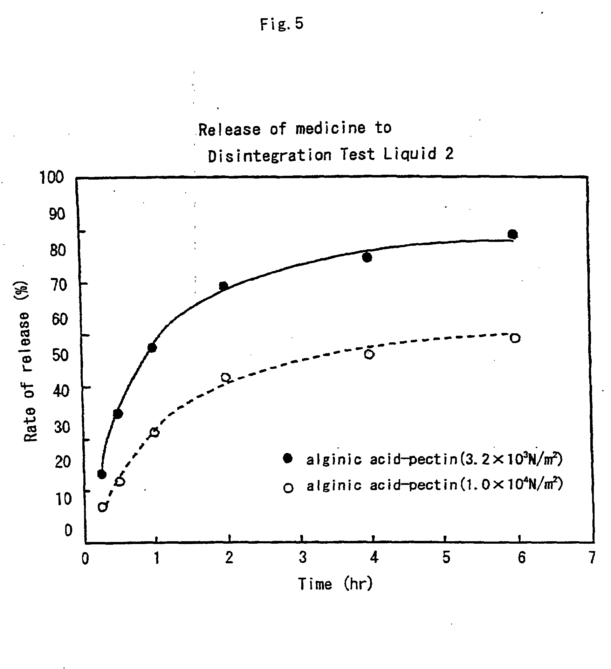

The two prepared liquid preparations were gelled by dipping for 6 hours in Japanese Pharmacopoeia Disintegration Test Liquid 1. The strength of the gel was 3.02×10³ N/m² for 0.5% alginic acid-0.5% pectin and 1.00×10⁴ N/m² for 1% alginic acid-1% pectin. The gel was removed, and the adhering Japanese Pharmacopoeia Disintegration Test Liquid 1 was removed by a filter paper. Then, the gel was dipped in Japanese Pharmacopoeia Disintegration Test Liquid 2, whereby a reproduction test was conducted wherein the riboflavin-liquid matrix was assumed to be transferred via the stomach into the small intestine. The release of riboflavin from the gel was measured in the same manner as in Example 2. The results are shown in Fig. 5.

As shown in Fig. 5, it was found that by selecting the water-soluble polymer as the constituent component or by combination thereof of the oral liquid preparation of the present invention, the physical strength and the disintegration of the gel can be regulated not only in the stomach but also in the small intestine, to demonstrate excellent sustained release.

### Example 7

Aqueous sodium alginate solutions prepared at various concentration (viscosity) were used in healthy 6 male adults as examinees, and an organoleptic test was conducted to examine easiness of administration. The results are shown in Table 3.

**Table 3**

| | | | | | | |
|---|---|---|---|---|---|---|
| Concentration of sodium alginate (wt%) | 1.0 | 1.2 | 1.4 | 1.6 | 1.8 | 2.0 |
| Viscosity (Pa·S) | 4.0×10⁻² | 6.0×10⁻² | 8.0×10⁻² | 1.0×10⁻¹ | 2.0×10⁻¹ | 5.0×10⁻¹ |
| Number of persons who can administer it | 6 | 6 | 6 | 6 | 5 | 0 |
| Number of persons who can administer it with slight difficulty | 0 | 0 | 0 | 0 | 1 | 4 |
| Number of persons who hardly administer it | 0 | 0 | 0 | 0 | 0 | 2 |

From the results in this example, it was revealed that in consideration of easiness of administration, the viscosity of the liquid matrix is preferably 3.0×10⁻¹ Pa·s or less.

### Example 8

20 ml of 1% aqueous sodium alginate solution into 50 mg of magnesium chloride had been added as bitter-taste component was used in healthy 6 male adults as examinees, and an organoleptic test was conducted to examine the masking effect on the bitter taste. As a comparative example, 20 ml of water containing 50 mg of magnesium chloride was used. The results are shown in Table 4.

**Table 4**

| Evaluation of masking effect on bitter taste | | |
|---|---|---|
| ⓞ | ○ | × |
| 4 persons | 2 persons | 0 person |

| | | |
|---|---|---|
| ⓞ; Signifiant relaxation of bitter taste | | |
| ○: Slight relaxation of bitter taste | | |
| ×: No relaxation of bitter taste | | |

From the results of this test, it was revealed that the liquid matrix of the present invention has a masking effect on the bitter taste of the medicine.

### Example 9

Gellan gum and LM pectin were dissolved in distilled water to prepare a liquid matrix containing each component at a concentration of 0.5%. 10 mg of riboflavin was added to and dissolved in 10 ml of this liquid matrix, to adjust the pH to 7.4.

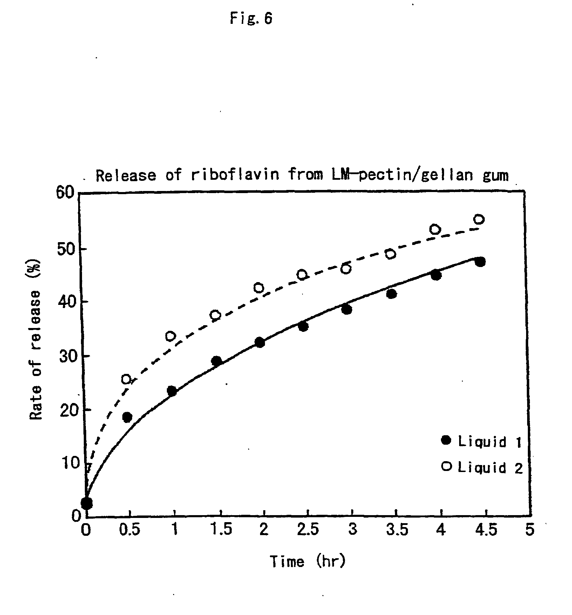

1 ml of the riboflavin-liquid matrix was dropped into Japanese Pharmacopoeia disintegration Disintegration Test Liquid 1, and a reproduction experiment was conducted wherein the riboflavin-liquid matrix was assumed to be transferred into the stomach. As a result, the dropped riboflavin-liquid matrix was gelled upon dropping. The strength of the gel was 3.00×10⁴ N/m². The release of riboflavin released from the gel was measured in the same manner as in Example 2. The release of the medicine into the Japanese Pharmacopoeia Disintegration Test Liquid 2 was also measured in the same manner as in Example 6. The results are shown in Fig. 6.

As shown in this result, the riboflavin in the gel was eluted gradually into the Disintegration Test Liquid 1 and 2 without being rapidly released. From this result, it was evidenced that the oral liquid preparation of the present invention shows excellent sustained release both in the stomach and in the small intestine.

### Example 10

The liquid preparations of the invention having compositions in Tables 5 to 9 were prepared.

**Table 5**

| | 1 | 2 | 3 | 4 | 5 | 6 | 7 |
|---|---|---|---|---|---|---|---|
| Amoxicillin | 0.75g | 0.5g | 0.5g | | 0.5g | | 0.5g |
| Clarithromycin | 0.2g | 0.2g | 0.2g | 0.3g | | | |
| Roxithromycin | | | | | | 0.15g | 0.15g |
| Pectin | 4g | 0.2g | | | | 0.02g | 0.01 g |
| Gellan gum | 1g | 0.05g | 0.03g | | | 0.04g | 0.03g |
| Sodium alginate | | | | 0.05g | 0.05g | | |
| Calcium carbonate | | | | 0.05g | 0.05g | | |
| White soft sugar | 20g | 1g | 1g | 1g | 1g | 1g | 1g |
| Distilled water | balance | balance | balance | balance | balance | balance | balance |
| Total amount | 200g | 10g | 10g | 10g | 10g | 10g | 10g |

**Table 6**

| | 8 | 9 | 10 | 11 | 12 | 13 |
|---|---|---|---|---|---|---|
| Cefaclor | 0.25g | | | 0.25g | | 0.25g |
| Cefalexin | | 0.5g | 0.5g | | 0.5g | |
| Amoxicillin | | | | | 0.5g | 0.5g |
| Clarithromycin | 0.2g | | | | | |
| Pectin | | 0.04g | 0.02g | 0.02g | | |
| Gellan gum | | | 0.05g | 0.05g | | |
| Sodium alginate | 0.04g | | | | 0.05g | 0.05g |
| Calcium carbonate | 0.03g | 0.04g | | | 0.05g | 0.05g |
| D-Sorbitol | 1g | 1g | 1g | 1g | 1g | 1g |
| Distilled water | balance | balance | balance | balance | balance | balance |
| Total amount | 10g | 10g | 10g | 10g | 10g | 10g |

**Table 7**

| | 14 | 15 | 16 | 17 | 18 |
|---|---|---|---|---|---|
| Ofloxacin | 0.2g | | | 0.2g | |
| Sparfloxacin | | 0.1g | | | |
| Tosufloxacin tosilate | | | 0.15g | | 0.15g |
| Amoxicillin | 0.5g | 0.5g | 0.5g | | |
| Clarithromycin | | | | 0.2g | 0.2g |
| Pectin | | 0.02g | 0.02g | | |
| Gellan gum | | 0.05g | 0.05g | | |
| Carrageenan | 0.05g | | | 0.05g | 0.05g |
| Calcium carbonate | 0.04g | | | 0.05g | 0.05g |
| D-Sorbitol | 1g | 1g | 1g | 1g | 1g |
| Distilled water | balance | balance | balance | balance | balance |
| Total amount | 10g | 10g | 10g | 10g | 10g |

**Table 8**

| | 19 | 20 | 21 | 22 | 23 | 24 |
|---|---|---|---|---|---|---|
| Minocycline HCl | | | 0.1g | | 0.1g | 0.1g |
| Nalidixic acid | | | | 1g | | |
| Norfloxacin | 0.2g | 0.2g | | | | |
| Clarithromycin | | | | | | |
| Amoxicillin | 0.5g | 0.5g | | 0.5g | | |
| Pectin | 0.01 g | 0.03g | 0.02g | 0.01 g | 0.01g | |
| Gellan gum | 0.03g | | 0.07g | 0.04g | 0.04g | |
| Sodium alginate | | 0.03g | | | | 0.05g |
| Calcium carbonate | | | | | | 0.05g |
| White soft sugar | 1g | 1g | 1g | 1g | 1g | 1g |
| Distilled water | balance | balance | balance | balance | balance | balance |
| Total amount | 10g | 10g | 10g | 10g | 10g | 10g |

**Table 9**

| | 25 | 26 | 27 | 28 | 29 |
|---|---|---|---|---|---|
| Levofloxacin | | 0.1g | 0.1g | | |
| Metronidazole | 0.25g | | | | 0.25g |
| Bismuth subcarbonate | | | | 1g | |
| Clarithromycin | | | 0.2g | | |
| Amoxicillin | 0.5g | 0.5g | | 0.5g | 0.5g |
| Pectin | 0.02g | 0.02g | 0.02g | | |
| Gellan gum | 0.05g | 0.05g | 0.05g | | |
| Sodium alginate | | | | 0.05g | 0.05g |
| Calcium carbonate | | | | 0.05g | 0.05g |
| D-Sorbitol | 1g | 1g | 1g | 1g | 1g |
| Distilled water | balance | balance | balance | balance | balance |
| Total amount | 10g | 10g | 10g | 10g | 10g |

When each of 1 ml liquid preparations shown in Nos. 2, 3, 6, 8, 9, 10, 14, 16, 21 and 26 in the tables was added gently to a Petri dish containing 10 mL artificial stomach fluid (the Japanese Pharmacopoeia Disintegration Test Liquid 1), all of liquid preparation gelled immediately upon contacting with the artificial stomach fluid.

The gelled preparation was dipped in 10 ml culture of H.pylori (CFU: 1×10⁷, 1×10⁸, 1×10⁹/ml, Brucella broth containing 10% inactivated horse serum) and cultured under slight aerobic conditions at 37°C for 24 hours by using Campy Pak (BBL). Similarly, only the gel not containing the antibacterial agent was used as a negative control, and 0.5 g of amoxicillin and 0.2 g of clarithromycin were used as positive controls, and these were added and cultured.

After 24 hours, each culture was sprayed onto M-BHM pylori agar medium containing 10% inactivated horse serum (K.K. Nikken Seibutsu Igaku Kenkyusho) and cultured at 37°C for 7 days, and then whether H. pylori proliferated or not was examined for evaluation. The determination of the antibacterial activity was evaluated as positive when the bacteria were eliminated or evidently reduced, and as negative (-) when the bacteria proliferated. The results are shown in Table 10 below.

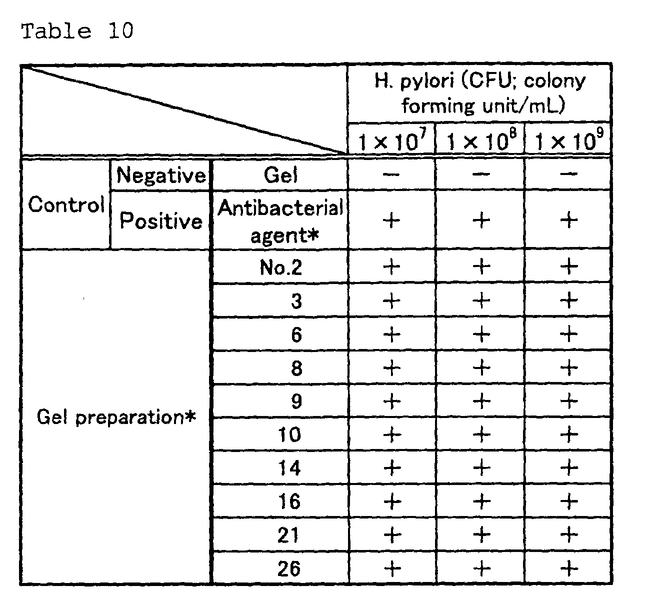

From the above result, it was revealed that the liquid preparation of the present invention gelled rapidly with stomach fluid, and indicated the same activity as by administration of the antibacterial agent as it was.

The antibiotic is generally unstable to strong acid, and upon oral administration, its activity is reduced with stomach acid. On the other hand, in the liquid preparation of the present invention, the antibacterial agent is protected with the gel, and could thus maintain the antibacterial activity even in the stomach in order to treat H. pylori infection.

### Example 11

First, according to a known method (Hirayama et al., Journal of Gastroenterogy, Vol. 31, pp. 755-757 (1996)), H. pylori (ATCC43504) was cultured under slight aerobic conditions in Brucella broth (BBL) containing 10% inactivated horse serum to prepare a culture of 1×10⁹ CFU (colony forming unit)/ml.

Then, male mongolian gerbil (Meriones unguiculatus; MGS/Sea, weight about 60 g), 3 to 4 animals/group, were fasted for 24 hours, and then 200 µl of the Helicobacter pylori culture solution (2×10⁸ CFU) was inoculated via an oral probe for rat into the stomach.

Seven days after inoculation, an antibacterial agent was administered twice (morning and evening) every day for 2 days according to a known method (Shimidzu et al., Cancer Research, Vol. 60, pp. 1512-1514 (2000)).

As the antibacterial agent, liquid preparation No. 23 in Example 10 (minocycline hydrochloride added as the antibacterial agent to the liquid matrix consisting of pectin and gellan gum) was administered. The dose in terms of the amount of the antibacterial agent administered was 17 mg/body weight in each administration.

As the control, 0.5% CMC solution only and a combined medicine of 3 agents, that is, insurance-applicable lansoprazole (proton pump inhibitor), amoxicillin (antibiotic) and clarithromycin (antibiotic) in a dose of 10, 3, 30 mg/body kg, respectively, were administered.

In judgment of microbial eradication, collected stomach mucous membrane tissues were cultured under slightly aerobic conditions in Brucella broth (BBL) medium containing 10% inactivated horse serum for 24 hours and then sprayed on M-BHM pylori agar medium containing 10% inactivated horse serum, to determine microbial eradication by detecting H. pylori.

In judgment of effectiveness of the medicines, the number of H. pylori bacteria detected in the stomach mucous membrane tissues of the sand rats given the combined medicine of 3 agents (this combined medicine is used in the method of eradicating the bacteria at present) is 0.001% (i.e. 1×10⁴ CFU/mL) relative to the original number of administered bacteria (1×10⁹ CFU/mL), and thus a number identical to or less than this number of bacteria was regarded as effective. The results are shown in Table 11.

**Table 11**

| | Medicine | Effective number of animals (%) |
|---|---|---|
| No. 23 | minocycline hydrochloride | 5/5(100) |
| CMC solution only | none | 0/5 ( 0) |
| Control of microbial eradication | combination of the 3 agents | 5/5(100) |

From the above result, the liquid preparation No. 23 in the present invention achieved the same level of microbial eradication as by therapy with combination of the 3 agents which is the present-day therapy of microbial eradication.

The liquid preparation No. 23 gelled at the surface layer of the stomach mucous membrane by stomach acid, whereby minocycline hydrochloride contained in the gel was protected against rapid hydrolysis by stomach acid, and a sufficient antibacterial effect was considered to be achieved by sustainedly releasing the medicine to the mucous membrane surface layer where H. pylori existed.

On the other hand, the administration of combination of 3 agents lead to reduction in H. pylori, but the amount of the combined 3 agents administered was very large. Therefore, there occurred a side effect that congestion was observed in all regions of digestive tracts from the small to large intestines. Particularly, the duodenum was swollen with congestion, and the gallbladder was enlarged. Further, possibly because of inhibition of stomach acid secretion by the proton pump inhibitor, the stomach was swollen significantly, and regions from the cardia to the esophagus underwent reddish inflammation.

However, when the liquid preparation of the present invention was administered, such side effect did not occur.

### Example 12

200 mL of liquid preparation containing 750 mg amoxicillin and 200 mg clarithromycin, according to No. 1 in Example 10, was administered every day before sleeping to each of 3 patients in their thirties to fifties who were judged to have gastritis by stomach endoscopic examination and judged to be H. pylori positive by a biopsy sample culture test. This administration was continued for 1 week. After the administration, whether H. pylori was present or absent was judged by an urea respiration test method. The results are shown in Table 12 below.

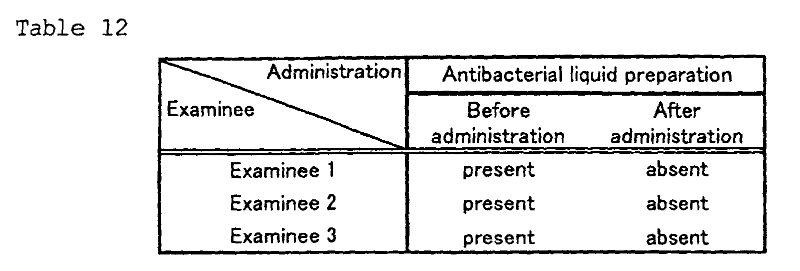

In the method of administering insurance-applicable 3 agents, that is, lansoprazole, amoxicillin and clarithromycin, 60 mg of lansoprazole, 1,500 mg of amoxicillin and 400 mg of clarithromycin shall be administered every day over 7 days. From the above result, it was revealed that lansoprazole as a proton pump inhibitor was not necessary for the oral liquid preparation of the present invention, and combined use of the 2 antibiotics was sufficient, and half of the above administration, that is, the daily dose of 750 mg amoxicillin and 200 mg clarithromycin indicated sufficient effect.

In addition, findings considered as side effects such as reflux esophagitis, diarrhea and taste abnormality, which are reported in the eradication of H. pylori, were not recognized in the examinees. Accordingly, the antibacterial agent consisting of the oral liquid preparation of the present invention was considered unlikely to cause severer side effects than before, and it was proved that the bacteria can be removed while the daily life can be maintained.

### Example 13

The liquid preparations of the present invention having the compositions in Tables 13 to 15 were prepared.

**Table 13**

| | | 30 | 31 | 32 | 33 |
|---|---|---|---|---|---|
| Medicines | Cimetidine | 0.4g | 0.2g | | |
| | Pirenzepine | | | 0.025g | |
| | Gefarnate | | | | 0.1g |
| | Pectin | 0.2g | | | |
| | Gellan gum | 0.05g | | | |
| | Sodium alginate | | 0.05g | | |
| | Carrageenan | | | 0.05g | 0.05g |
| | Tamarind seed gum | | | 0.1g | |
| | Calcium carbonate | | 0.05g | 0.04g | 0.05g |
| | White soft sugar | | | 1.5g | |
| | D-Sorbitol | 2g | 2g | | 1g |
| | Distilled water | balance | balance | balance | balance |
| | Total amount | 10g | 10g | 10g | 10g |

**Table 14**

| | | 34 | 35 | 36 | 37 |
|---|---|---|---|---|---|
| Medicines | Urogastrone | 0.024g | | | |
| | Azulene sulfonate Na | | 0.445g | 0.99g | |
| | L-glutamine | | 0.0015g | 0.003g | |
| | Teprenone | | | | 0.05g |
| | Pectin | 0.2g | | 0.2g | 0.2g |
| | Gellan gum | 0.05g | | 0.08g | |
| | Sodium alginate | | 0.05g | | 0.05g |
| | Carrageenan | | 0.05g | | |
| | Tamarind seed gum | | | | 0.1 g |
| | Calcium carbonate | | 0.04g | | |
| | White soft sugar | | | 2g | |
| | D-Sorbitol | 1g | 1g | | 1.5g |
| | Distilled water | balance | balance | balance | balance |
| | Total amount | 10g | 10g | 10g | 10g |

**Table 15**

| | | 38 | 39 | 40 | 41 |
|---|---|---|---|---|---|
| Medicines | Cetraxate HCl | 0.2g | | | |
| | Sulpiride | | 0.2g | 0.1g | |
| | Alprostadil alfadex | | | | 0.0003g |
| | Pectin | 0.02g | | 0.02g | 0.04g |
| | Gellan gum | | 0.05g | | 0.05g |
| | Sodium alginate | 0.05g | | | |
| | Carrageenan | | | 0.04g | |
| | Tamarind seed gum | | | 0.05g | 0.1g |
| | Xanthane gum | | | | 0.01 g |
| | Calcium carbonate | 0.04g | | 0.05g | |
| | White soft sugar | 1g | 1g | 1.5g | |
| | D-Sorbitol | | | | 2g |
| | Distilled water | balance | balance | balance | balance |
| | Total amount | 10 g | 10 g | 10 g | 10 g |

### Example 14: Test of therapeutic effect on a rat as acetic acid chronic ulcer model

24 Wister male rats weighing about 200 g were divided into 8 groups and fasted overnight, and acetic acid ulcer was causeed in each rat. That is, the rats were anesthetized under Nembutal and the belly was cut along the midline to remove the stomach. Then, a cylinder of 100 mm in diameter charged with absorbent cotton impregnated sufficiently with 100% acetic acid was pressed against the border between the stomach and the pyloric region for about 30 seconds, and the acetic acid adhering to the serous membrane was wiped off with a germ-free gauze, and the belly was sutured. After 6 weeks, one rat was picked up as the control at random from the untreated group, killed and dissected to confirm ulcer formation.

0.5 ml of cetraxate hydrochloride liquid preparation No. 38, the cimetidine liquid preparation No. 30 and the teprenone liquid preparation No. 37, respectively, were administered daily once for 2 weeks to the stomachs of 3 rats as liquid preparation administration group. As the control, a purified aqueous solution of each medicine was administered to the 3 groups each consisting of 3 rats (positive control), the liquid preparation consisting of the liquid matrix only was administered to one group of 3 rats (negative control), and purified water only was administered to one group of 3 rats (untreated group).

In the seventh and eight weeks, one untreated rat selected at random was killed and the belly was opened to reconfirm formation of stomach ulcer. Each rat was killed in the eight week and the area of the ulcer was measured, and the ulcer coefficient was determined according to a method of Okabe et al. (Okabe S, Roth JLA, Pfeitter CJ: A method for experimental penetrating gastric and duodenal ulcers in rats. Observation on normal healing. Amer J Dig Dis 16: 277-284, 1971). The ulcer coefficient was 1 for 1 to 10 mm², 2 for 11 to 20 mm², and 3 for 21 to 30 mm². The results are shown in Table 16.

**Table 16**

| | | Ulcer coefficient | | | Average ulcer coefficient |
|---|---|---|---|---|---|
| Untreated group | | 3 | - | - | - |
| No. 38 | | 1 | 2 | 1 | 1.33 |
| Only liquid matrix | Negative control | 3 | 2 | 3 | 2.67 |
| Cetraxate HCl solution | Positive control | 2 | 2 | 3 | 2.33 |
| No. 30 | | 2 | 2 | 2 | 2 |
| Cimetidine solution | Positive control | 3 | 3 | 2 | 2.67 |
| No. 37 | | 2 | 2 | 1 | 1.67 |
| Teprenone sotution | Positive control | 3 | 2 | 2 | 2.33 |

From the above results, it was proved that the liquid preparation according to the present invention exhibits therapeutic effect on stomach ulcer. Teprenone having tissue-repairing action or cetraxate hydrochloride having viscous fluid production/secretion promoting action were recognized to be more effective against stomach ulcer than by attach factor inhibiting type such as cimetidine.

On the other hand, even if the medicine solution was administered directly (positive control), effect of reducing ulcer was recognized. However, the effect was lower than that of the liquid preparation of the present invention.

Accordingly, it was proved that the liquid preparation of the present invention exhibits a higher therapeutic effect on ulcer than that of the conventional preparation.

### Example 15: Therapeutic effect of liquid preparation in indomethacin ulcer rat model

18 Wister male rats weighing about 200 g were divided into 4 groups, that is, an experimental group of 5 animals, control groups (a positive control group of 5 animals and a negative control group of 5 groups), and an untreated group for confirming formation of ulcer. These rats were fasted for 24 hours, and then indomethacin (IND) suspended in 1% carboxymethyl cellulose (CMS) solution was administered in a dose of 20 mg/kg to the rats via an oral probe for rat into the stomach to induce indomethacin ulcer. In a preliminary test, the blood concentration of indomethacin reached the maximum after 3 hours, and erosive bleeding was observed in the stomach.

3 hours after administration of indomethacin, prostaglandin (PGE1) liquid preparation No. 41 (containing alprostadil alfadex as the medicine), PGE1 solution dissolved in purified water in a dose of 3.3 µg/kg in weight as a positive control, the liquid matrix only was administered as a negative control, and purified water only for confirming formation of ulcer to the untreated group, respectively, was administered. After 1 hour, the rats were given feed. The untreated group after 3 hours from administration of indomethacin and the other groups after 24 hours were killed, and dissected to judge ulcer and bleeding in the stomach.

Indomethacin ulcer in the fasted rats is different from that in humans, and develops in the stomach without generating in the pyloric vestibule. Therefore, judgment was made on the basis of bleeding. That is, no bleeding was expressed as negative (-), trace bleeding was given (±), dotted bleeding (+), and broad bleeding (++). The results are shown in Table 17.

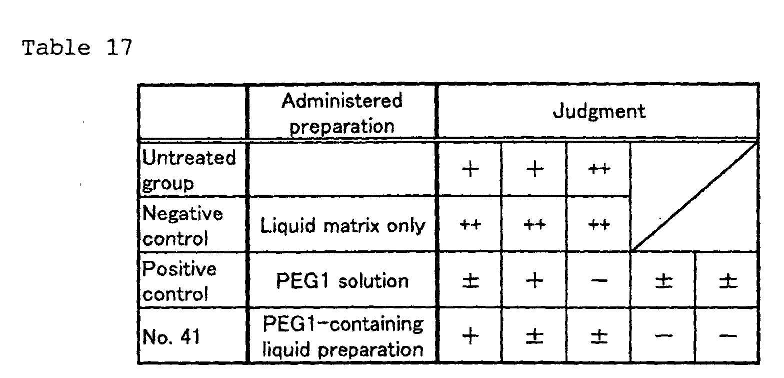

It is known that in an indomethacin ulcer model using a fasted rat, bleeding erosion in the stomach is inhibited by H2 blocker, acid regulator, anti-choline agent, proton pump inhibitor, prostaglandin preparation or mucous membrane protecting agent (Susumu Okabe, Experimental Model of Digestive Ulcer (in Japanese), Nippon Rinsho, 42: 43-47, 1979). On the other hand, in the rat fasted for 24 hours and then fed for only 1 hour, the lesion in the corpus ventriculi is inhibited, and similar to human ulcer, the ulcer develops in regions of the pyloric vestibule to the small intestine. It is also known that no preparation other than the prostaglandin preparation is effective against this ulcer in the pyloric vestibule (Satoh, H., et al., Gastroenterology, 81: 719-725, 1981)).

From the above result, it was revealed that in the indomethacin ulcer rat fasted for 24 hours in this experiment, bleeding was ameliorated by administering prostaglandin (PEG1) having a site protection effect on the stomach mucous membrane.

However, it was revealed that the effect of PEG1 is higher when administered as the liquid preparation of the present invention than by administering as it is. This suggests that the liquid matrix in the liquid preparation of the present invention gels with stomach acid thereby protecting the stomach mucous membrane physically and sustainedly releasing the medicine (EG1 in this example), whereby PEG1 is utilized more effectively in the affected area to treat the ulcer.

| Preparation Example 1 | |
|---|---|
| Ingradients | Blending quantity (mass%) |
| Sodium alginate | 0.5 |
| Pectin | 0.5 |
| Calcium carbonate | 5.0 |
| Methyl paraben | 0.3 |
| Distilled water | suitable amount |

| Preparation Example 2 | |
|---|---|
| Ingradients | Blending quantity (mass%) |
| Sodium alginate | 0.5 |
| Pectin | 0.5 |
| Calcium carbonate | 5.0 |
| Distilled water | suitable amount |

| Preparation Example 3 | |
|---|---|
| Ingradients | Blending quantity (mass%) |
| Gellan gum | 1.0 |
| Arabic gum | 1.0 |
| Distilled water | suitable amount |

| Preparation Example 4 | |
|---|---|
| Ingradients | Blending quantity (mass%) |
| κ-Carrageenan | 0.5 |
| Magnesium oxide | 5.0 |
| Distilled water | suitable amount |

| Preparation Example 5 | |
|---|---|
| Ingradients | Blending quantity (mass%) |
| Sodium alginate | 0.5 |
| Pectin | 0.5 |
| Sawacillin (Amoxicillin) | 0.5 |
| Calcium carbonate | 5.0 |
| Methyl paraben | 0.3 |
| Distilled water | suitable amount |

| Preparation Example 6 | |
|---|---|
| Ingradients | Blending quantity (mass%) |
| Sodium polyacrylate | 1.0 |
| Aluminum hydroxide | 5.0 |
| Distilled water | suitable amount |

| Preparation Example 7 | |
|---|---|
| Ingradients | Blending quantity (mass%) |
| Gellan gum | 1.0 |
| Amoxicillin | 2.5 |
| Calcium carbonate | 5.0 |
| Glycerin | 20.0 |
| Preservative | suitable amount |
| Distilled water | suitable amount |

| Preparation Example 8 | |
|---|---|
| Ingradients | Blending quantity (mass%) |
| Cimetidine | 2.0 |
| Sodium alginate | 0.5 |
| Pectin | 0.5 |
| Calcium carbonate | 5.0 |
| D-sorbitol | 20.0 |
| pH adjusting agent (hydrochloric acid) | suitable amount |
| Distilled water | suitable amount |

| Preparation Example 9 | |
|---|---|
| Ingradients | Blending quantity (mass%) |
| Diclofenac sodium | 0.5 |
| Sodium alginate | 0.5 |
| Pectin | 0.5 |
| Calcium carbonate | 5.0 |
| Distilled water | suitable amount |

| Preparation Example 10 | |
|---|---|
| Ingradients | Blending quantity (mass%) |
| Theophylline | 2.0 |
| Sodium alginate | 0.5 |
| Pectin | 0.5 |
| Calcium carbonate | 5.0 |
| Preservative | suitable amount |
| Distilled water | suitable amount |

| Preparation Example 11 | |
|---|---|
| Ingradients | Blending quantity (mass%) |
| Ketoprofen | 2.0 |
| Sodium alginate | 0.5 |
| Pectin | 0.5 |
| Calcium carbonate | 5.0 |
| Propylene glycol | 20.0 |
| Distilled water | suitable amount |

### Exploitation in Industry

The liquid matrix of the present invention can easily solubilize, disperse or suspend medicine, is liquid to permit easy swallowing, is easily and highly operative in sterilization, has effect of masking bitter tastes of medicine and the like, and can gel in the living body to regulate the rate of release of the medicine. Accordingly, an oral liquid preparation containing medicine in the liquid matrix of the present invention can achieve nonconventional excellent sustained release although it is liquid.

In particular, the oral liquid preparation mixed with efficacy component showing anti-H. pylori activity as the medicine gels by introducing it into the stomach, and thus it protects the efficacy component unstable to acidic conditions and is disintegrated sustainedly to release the efficacy component, whereby the content of the efficacy component contained therein and the frequency of administration can be reduced, and side effects hardly occur.

In addition, the liquid preparation does not necessitate a proton pump inhibitor and the like for inhibiting secretion of stomach acid, and thus there is no side effect of such medicine.

Unlike conventional anti-H. pylori preparations, the liquid preparation of the present invention applied to therapy for H. pylori infection has a particularly excellent feature that proton pump inhibitors are not required, and thus it is extremely effective as a therapeutic agent for Helicobacter pylori infection.

The oral liquid preparation mixed with the medicine having therapeutic effect on stomach ulcer or duodenal ulcer also shows sustained release of the medicine and exhibits a particularly high therapeutic effect. That is, the oral liquid preparation unlike the conventional anti-ulcer agent, can sustainedly release the medicine directly to an affected region, and thus the effectiveness of the medicine is high, and side effects can be reduced.

Accordingly, the liquid preparation of the present invention used in therapy of stomach ulcer or duodenal ulcer can reduce its dose as compared with the conventional anti-ulcer agent, and can thus exhibit a high therapeutic effect with less side effects, and is thus extremely useful.

## Claims

1. A liquid matrix which is a liquid assistant for facilitating swallowing medicine **characterized in** comprising a water-soluble polymer gelling under acidic conditions, and the breaking stress of the gel is about 3.00×10² N/m² or more.

2. The liquid matrix according to claim 1, wherein the breaking stress of the gel is 2.00×10³ N/m² or more.

3. The liquid matrix according to claim 1 or 2, wherein the viscosity of the liquid matrix is 3.0×10⁻¹ Pa·s or less.

4. The liquid matrix according to any one of claims 1 to 3, comprising insoluble salt releasing polyvalent metallic cation under acidic conditions.

5. The liquid matrix according to claim 4, wherein the insoluble salt is alkaline earth metal salt of inorganic acid.

6. The liquid matrix according to any one of claims 1 to 5, wherein the water-soluble polymer has carboxyl group and/or sulfonic acid group in the chemical structure thereof.

7. The liquid matrix according to any one of claims 1 to 5, wherein the water-soluble polymer is alginate.

8. The liquid matrix according to any one of claims 1 to 5, wherein the water-soluble polymer is pectin.

9. The liquid matrix according to any one of claims 1 to 5, wherein the water-soluble polymer is combination of alginic acid and pectin.

10. The liquid matrix according to any one of claims 1 to 5, wherein the water-soluble polymer is a combination of alginate and pectin.

11. The liquid matrix according to any one of claims 1 to 5, wherein the water-soluble polymer is gellan gum.

12. The liquid matrix according to any one of claims 1 to 5, wherein the water-soluble polymer is combination of gellan gum and pectin.

13. The liquid matrix according to any one of claims 1 to 12, wherein the viscosity of the liquid matrix is about 1.0×10⁻¹ Pa·s or less.

14. An oral liquid preparation **characterized in** comprising the liquid matrix according to any one of claims 1 to 13 and medicine.

15. The oral liquid preparation according to claim 14, wherein the medicine has anti-Helicobacter *pylori* activity.

16. The oral liquid preparation according to claim 14 or 15, wherein the medicine is at least one member selected from the group consisting of penicillin antibiotics, macrolide antibiotics, tetracycline antibiotics, cepham antibiotics, and pyridonecarboxylic acid synthetic antibacterial agents.

17. The oral liquid preparation according to claim 16, wherein the medicine is at least one member selected from the group consisting of amoxicillin, clarithromycin, roxithromycin, minocycline hydrochloride, cephaclor, cephalexin, ofloxacin, tosufloxacin tosylate, and levofloxacin.

18. The oral liquid preparation according to any one of claims 14 to 17, wherein the liquid matrix is gelled in the stomach thereby exhibiting sustained release of the medicine.

19. The oral liquid preparation according to claim 14, wherein the medicine has therapeutic effect on stomach ulcer or duodenal ulcer.

20. The oral liquid preparation according to claim 19, wherein the medicine having therapeutic effect on stomach ulcer or duodenal ulcer has effect of promoting protection factor.

21. The oral liquid preparation according to claim 20, wherein the medicine having an effect of promoting protection factor and a therapeutic effect on stomach ulcer or duodenal ulcer is prostaglandin.

22. The oral liquid preparation according to any one of claims 19 to 21, wherein the liquid matrix is gelled in the stomach thereby exhibiting sustained release of the medicine.

23. A method **characterized in** utilizing an aqueous solution of a water-soluble polymer gelling under acidic conditions as a component in a sustained-release oral liquid preparation.

## Amended claims

### Amended claims under Art. 19.1 PCT

[Received on July 22, 2003 by the International Office; new claim 18 was added; the originally filed claim 16 was amended; the originally filed claims 18 to 23 were amended into the amended claims 19 to 24; no other claims were changed (2 pages)]

10. The liquid matrix according to any one of claims 1 to 5, wherein the water-soluble polymer is a combination of alginate and pectin.

11. The liquid matrix according to any one of claims 1 to 5, wherein the water-soluble polymer is gellan gum.

12. The liquid matrix according to any one of claims 1 to 5, wherein the water-soluble polymer is combination of gellan gum and pectin.

13. The liquid matrix according to any one of claims 1 to 12, wherein the viscosity of the liquid matrix is about 1.0× 10⁻¹ Pa·s or less.

14. An oral liquid preparation **characterized in** comprising the liquid matrix according to any one of claims 1 to 13 and medicine.

15. The oral liquid preparation according to claim 14, wherein the medicine has anti-*Helicobacter pylori* activity.

16. (Amended) The oral liquid preparation according to claim 14 or 15, wherein the medicine is at least one member selected from the group consisting of penicillin antibiotics, macrolide antibiotics, tetracycline antibiotics, cepham antibiotics, and pyridonecarboxylic acid synthetic antibacterial agents and metronidazole.

17. The oral liquid preparation according to claim 16, wherein the medicine is at least one member selected from the group consisting of amoxicillin, clarithromycin, roxithromycin, minocycline hydrochloride, cephaclor, cephalexin, ofloxacin, tosufloxacin tosylate, and levofloxacin.

18. (Added) The oral liquid preparation according to claim 16, wherein the medicine is metronidazole.

19. (Amended) The oral liquid preparation according to any one of claims 14 to 18, wherein the liquid matrix is gelled in the stomach thereby exhibiting sustained release of the medicine.

20. (Amended) The oral liquid preparation according to claim 14, wherein the medicine has effect of promoting protection factor.

21. (Amended) The oral liquid preparation according to claim 20, wherein the medicine having therapeutic effect on stomach ulcer or duodenal ulcer is of protection factor promoting type.

22. (Amended) The oral liquid preparation according to claim 21, wherein the protection factor promoting type medicine having a therapeutic effect on stomach ulcer or duodenal ulcer is prostaglandin.

23. (Amended) The oral liquid preparation according to any one of claims 20 to 22, wherein the liquid matrix is gelled in the stomach thereby exhibiting sustained release of the medicine.

24. (Amended) A method **characterized in** utilizing an aqueous solution of a water-soluble polymer gelling under acidic conditions as a component in a sustained-release oral liquid preparation.
